# EUROPEAN PATENT APPLICATION

(11) **EP 4 166 649 A1**
(43) Date of publication of application: **19.04.2023**
(21) Application number: 21823134.8
(22) Date of filing: 21.04.2021
(51) Int. Cl.: C12N 1/19, C07K 14/165, C12N 15/50, C12N 15/81, A61K 39/215, A61K 9/00, A61P 37/04, A61P 31/14

(54) **ORAL RECOMBINANT YEAST FOR EXPRESSING S PROTEIN OF NOVEL CORONAVIRUS, PREPARATION THEREFOR, AND APPLICATION THEREOF**

(30) Priority: 11.06.2020 CN 202010529961
(71) Applicant: Tianjin University, Tianjin 300072 (CN)
(72) Inventor: HUANG, Jinhai, Tianjin 300072 (CN); ZHANG, Lilin, Tianjin 300072 (CN); GUO, Yanyu, Tianjin 300072 (CN)
(74) Representative: Cabinet Beau de Loménie
(86) International application number: PCT/CN2021/088592
(87) International publication number: WO 2021/249031

(57) **Abstract**

The present invention discloses an oral recombinant yeast for expressing an S protein of a novel coronavirus and preparation and application thereof. The oral recombinant yeast contains 16 to 1035 amino acids of the S protein, and contains an RBD domain and an FP fusion peptide. A complete transcriptional unit *GPD-S(RBD-FP)-TU* of a truncated S protein constructed in vitro is integrated into a yeast genome through homologous recombination, the S protein is displayed on a surface of a yeast cell by an Aga1-Aga2 surface display system; a S protein surface display type recombinant yeast strain ST1814G-S(RBD-FP) is obtained, and the obtained strain is used for preparing the oral recombinant yeast.

## Description

### SUMMARY OF THE INVENTION

The present invention belongs to the technical field of biological genetic engineering, and relates to an oral recombinant yeast for expressing an S protein of novel Coronavirus and preparation and application thereof.

### BACKGROUNDART

Corona Virus Disease 2019 (COVID-19) is an acute respiratory system infectious disease caused by infection with Severe Acute Respiratory Syndrome Coronavirus 2 (SARS-CoV-2). Since December, 2019, some cases of pneumonia of unknown cause have been consecutively found in some hospitals in Wuhan, Hubei Province. Such pneumonia was characterized by sustained human-to-human transmission, and has gradually spread to more than 200 countries and regions around the world.

The SARS-CoV-2 is a single-stranded positive-stranded RNA virus, which is classified as a member of Subfamily *Orthocoronavirinae,* Family *Coronaviridae,* and Order *Nidovirales.* It belongs to beta coronaviruses along with Severe Acute Respiratory Syndrome (SARS) and Middle East Respiratory Syndrome (MERS). A length of a genome is 29,903 bp, encoding main structural proteins that include Spike glycoprotein (S), Envelop (E), nucleocapsid phosphoprotein (N), membrane glycoprotein (M) and the like. Spike protein S in SARS-CoV and MERS-CoV binds to host cells by different receptor-binding domains (RBDs). The S protein of the MERS-CoV binds to dipeptidyl peptidase 4 (DPP-4, also known as CD26 ^{[1]}) of the host cells, the SARS-CoV is consistent with the SARS-CoV-2, and Angiotensin converting enzyme 2 (ACE2) on the surfaces of the cells is a binding site of the RBD of the S protein ^{[2,3]}. Studies have shown that RBD domains of the SARS-CoV and the SARS-CoV-2 mainly differ in 318-507 aa at a C-terminal^{[4]}, and both of them do not have complete protection, so it is necessary to develop specific protective preparations against the SARS-CoV-2.

Mature exogenous protein expression systems include yeast cells, prokaryotic expression systems and baculovirus insect cell expression systems. Compared with the latter two elements, the yeast cells have the advantages of mature post-translational modification capacity, short culture cycle, safety, convenience, low production cost and the like ^{[5]}. Exogenous proteins are displayed on the surfaces of the yeast cells by using yeast surface display (YSD), so as to prepare oral protective preparations. Meanwhile, yeast cell wall polysaccharides have a regulatory effect on organism's immune systems, and can achieve an antiviral effect, enhance immune functions and promote the development of immune organs. Therefore, the development of the oral yeast preparations has a good application prospect.

### BRIEF SUMMARY OF THE INVENTION

One of the objectives of the present invention is to provide surface display type recombinant yeast for spike protein S of coronavirus and application thereof. Specifically, the recombinant yeast is used for the preparation of oral preparations.

The second objective of the present invention is to provide a preparation method of the oral recombinant yeast.

The third objective of the present invention is to provide application of the oral recombinant yeast.

The objectives of the present invention are achieved by the following technical solutions:
A recombinant yeast for expressing an S protein of coronavirus, ST1814G-S (RBD-FP), contains amino acids of the S protein at positions 16 to 1035.

A truncated S protein of the coronavirus is preferably amino acids at positions 300 to 1000, with a sequence characteristic of SEQ ID No. 1, containing an RBD domain at positions 330 to 521 and fusion peptides (FP) at positions 816 to 833.

A gene for expressing the truncated protein, contains an RBD domain of the spike protein S, namely basic groups at positions 991 to 1563 and an FP domain, namely basic groups at positions 2449 to 2499, with a nucleotide sequence of SEQ ID No. 2.

A plasmid *GPD-S(RBD-FP)-*TU of the recombinant yeast is constructed, which has a sequence characteristic of SEQ ID No. 3 and consists of the gene fragments according to claim 3 and a POT-GPD-TU vector.

A method for preparing the recombinant yeast for the S protein of the coronavirus includes the steps: integrating a complete transcriptional unit GPD*-S(RBD-FP)-*TU of the truncated S protein constructed in vitro into a yeast genome through homologous recombination, displaying the S protein on a surface of a yeast cell by an Aga1-Aga2 surface display system to obtain an S protein surface display type recombinant yeast strain ST1814G-S(RBD-FP), and preparing the oral recombinant yeast by using the obtained strain.

The method specifically includes the following steps:
(1) PCR amplification of an encoding gene of the spike protein S of the SARS-CoV-2: synthesizing an S gene by referring to a virogene sequence NC_045512.2 of the SARS-CoV-2, with a sequence characteristic of SEQ No. 2; and designing primers to amplify the encoding gene *S(RBD-FP)* of the S protein for yeast vector linkage with a plasmid pcDNA3.1-CoV-S as a template;
(2) tandem between an Aga2 gene and an encoding sequence *S(RBD-FP)* of the spike protein S of the SARS-CoV-2: linearizing the POT-GPD-TU vector by single enzyme digestion with *Bam*HI*,* seamlessly cloning and linking the S gene fragments to a surface display expression vector GPD-POT-TU to obtain the recombinant plasmid *GPD-S(RBD-FP*)-TU with a sequence characteristic of SEQ No. 3, transforming the recombinant plasmid into *E.coli* DH5a, and performing PCR and sequencing validation by using S gene test primers to obtain a positive clone; and
(3) construction of an S protein recombinant yeast strain: performing enzyme digestion and splicing on the recombinant plasmid GPD-*S(RBD-FP)*-TU, homologous arms URRs and an encoding sequence of a screening tag Leu to obtain a complete recombinant gene containing an S gene sequence, transforming the recombinant gene into a saccharomyces cerevisiae genome, obtaining a recombinant strain after screening with an auxotrophy type plate, testing a gene level by using the test primers, and validating a protein expression level by Western blot and immunofluorescence.

Disclosed is application of the recombinant yeast for the S protein of the coronavirus in preparation of a drug against the coronavirus.

The present invention has the beneficial effects that the truncated S protein surface display type oral recombinant yeast preparation containing a receptor-binding domain and an FP domain related to virus pathogenesis is prepared based on the spike protein S that triggers the initiation of infestation when the coronavirus binds to an ACE2 receptor of a host cell. Organism's protective immune responses are stimulated in an oral route, and with the help of the regulatory effect of yeast cell wall polysaccharides on organism's innate immune systems, more effective immunoprotection is achieved. Compared with novel adenovirus vaccines and mRNA vaccines, the oral recombinant yeast preparation is low in cost, capable of achieving large-scale scaled-up production, and safe and reliable, has a good application and development prospect, and provides options for immunologic prevention and control of the coronavirus.

The oral recombinant yeast preparation for the S protein of the coronavirus in the present invention is reported in China for the first time, and construction of the surface display strain and preparation of the preparation have certain innovativeness, so that a new thought and preparation are provided for prevention and control of COVID-19.

### BRIEF DESCRIPTION OF THE DRAWINGS

FIG. 1 is a structural pattern diagram of an encoding gene of an S protein of coronavirus;
FIG. 2 shows a PCR amplification result of a gene S(RBD-FP);
FIG. 3 shows a transformant test after a plasmid GPD-S(RBD-FP)-TU is transformed into *Escherichia coli;* and lanes 1-10 respectively represent PCR test results of colonies of different *Escherichia coli* transformants, CK+ is amplification with a PCR product of the gene *S(RBD-FP)* as a template, and CK- is a ddH₂O control;
FIG. 4 is a splicing pattern diagram of a complete transcriptional unit *GPD-S(RBD-*FP)-TU;
FIG. 5 shows a growth condition of *GPD-S(RBD-FP)-TU* completing yeast transformation in an SD-leu medium;
FIG. 6 shows genotype validation of a recombinant yeast ST1814G-S(RBD-FP); and genotypes of the transformed yeast corresponding to transformants No. 2 and No. 3 of *Escherichia coli* are validated. Lanes 1-6 are 6 different yeast transformants respectively, CK+ is PCR amplification with a plasmid *GPD-S(RBD-FP)-TU* as a template, and CK- is a negative control with ddH₂O as a template.
FIG. 7 shows Western blot validation of a yeast strain ST1814G-S(RBD-FP), and two bands are shown after a truncated S protein is tested by a His antibody. The protein with a large molecular weight is a glycosylation-modified truncated S protein, and the protein with a small molecular weight is a product obtained after digestion of protease; and lanes 3-1, 3-4 and 3-6 correspond to 3 different yeast transformants.
FIG. 8 shows immunofluorescence observation of a protein S(RBD-FP) in a recombinant yeast ST1814G-S(RBD-FP); and a blank strain ST1814G is taken as a control.
FIG. 9 shows a growth curve of a recombinant yeast ST1814G-S(RBD-FP) and its relationship with a protein expression trend; a shows a growth curve of bacteria, ST1814G is taken as a control, and the growth trend of the strain for expressing protein is consistent with that of the control group; and b shows analysis of protein expressions at different culture time points, and lanes 1-5 represent culture for 1 day to 5 days respectively.
FIG. 10 shows a test of specific IgA and IgG in serum of BALB/c mice orally taking a recombinant yeast; A shows serum IgG detection of mice in a non-oral group (Blank), a group orally taking an original blank yeast (Host) and a group orally taking a recombinant yeast (FP) in ELISA; and B shows serum IgA detection of the mice in the non-oral group (Blank), the group orally taking the original blank yeast (Host) and the group orally taking the recombinant yeast (FP) in ELISA.

### DETAILED DESCRIPTION OF THE PRESENT INVENTION

The present invention will be further explained below with reference to embodiments, the content of which should not be constituted to limit the scope of the present invention. Unless other specified, reagents in the embodiments are all commercial reagents.

### Embodiment 1. Construction of vector GPD-S(RBD-FP)-TU

### (1) Amplification of gene S(RBD-FP)

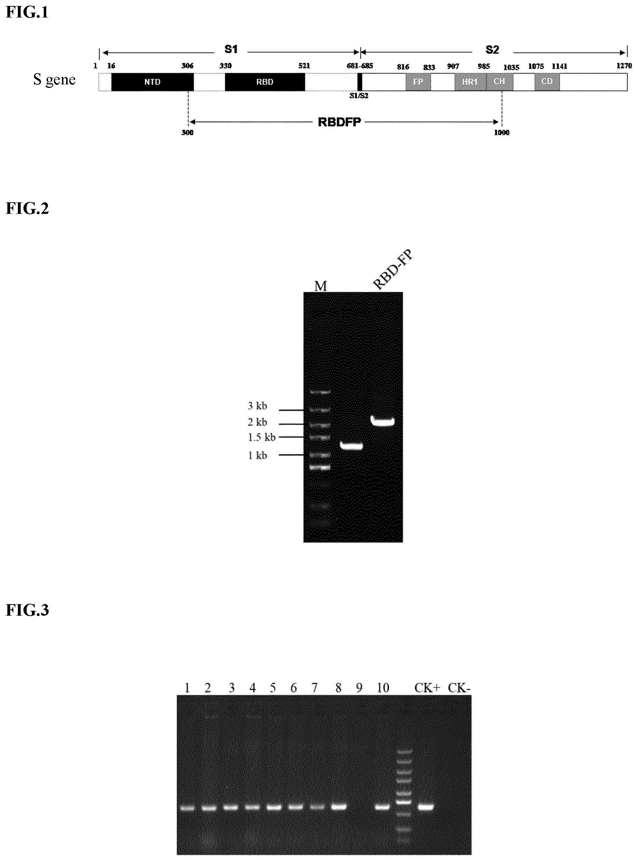

An encoding gene of an S protein consists of a subunit S1 and a subunit S2. The subunit S1 contains an RBD domain which can bind to a peptidase domain (PD) of an ACE2 receptor of a host cell; and the subunit S2 contains a hydrophobic fusion peptide (FP) sequence, which assists a virus in drawing close to and fusing with a host cell membrane, and similar structures also exist in SARS-CoV^{[8, 9]}. Primers CoV-S-F (SEQ ID NO. 4: GACGATAAGGTACCAGGATCCATGAAGTGTACGTTGAAATCCT) and CoV-S-R (SEQ ID NO. 5: gaattccaccacactggatccTCTGCCTGTGATCAACCTAT) were designed and synthesized according to an S gene sequence structure (shown in FIG. 1), gene fragments of the S protein were artificially synthesized according to a virus genome sequence (Genbank No.: MT407658.1) which has been reported, a plasmid pcDNA3.1-CoV-S was constructed, and the encoding gene *S(RBD-FP)* of the S protein containing RBD and an FP domain was amplified with the plasmid as a template. A PCR amplification system was as follows:

| | |
|---|---|
| Template DNA | 1 µL |
| CoV-S-F (10 µM) | 1 µL |
| CoV-S-R (10 µM) | 1 µL |
| 2^{∗}Hieff Mix (YEASEN) | 12.5 µL |
| ddH₂O | To 25 µL |

Amplification was performed by using the following PCR procedures:

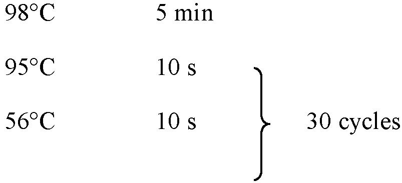

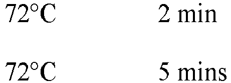

A length of a PCR product was 2106 bp.

A PCR result was shown in FIG. 2, and a lane on the rightmost side was an amplification result of the gene *S(RBD-FP).*

### (2) Construction of vector GPD-S(RBD-FP)-TU

The gene *S(RBD-FP)* was linked to a vector POT-GPD-TU constructed in a laboratory ^{[7]}. The vector POT-GPD-TU was linearized by enzyme digestion with *Bam*HI and then linked with the gene *S(RBD-FP),* an N-terminal of the gene *S(RBD-FP)* was linked with Aga2 in tandem for fusion expression, and a C-terminal of the gene had a His tag on the vector. A linked product was obtained by using a seamless clone kit (C112-01, Vazyme), and *E.coli*DH5 was transformed α. *Escherichia coli* transformants were screened with an Amp resistant plate, and S gene testing primers (S-F 331: aatattacaaacttgtgccct (SEQ ID NO. 6) and S-R 524: aacagttgctggtgcatgtag (SEQ ID NO. 7)) were used to perform PCR validation and sequencing.

Results were shown in FIG. 3, a length of a PCR amplification product was 579 bp, positive transformants were No. 1-8 and No. 9, the transformants No. 2 and 3 were selected for sequencing, and the results were consistent with an expected result, indicating that the plasmid *GPD-S(RBD-FP)*-TU was successfully constructed.

### Embodiment 2. Construction and test of recombinant yeast strain ST1814G-S(RBD-FP) of coronavirus

### (1) Construction of yeast transformation fragments

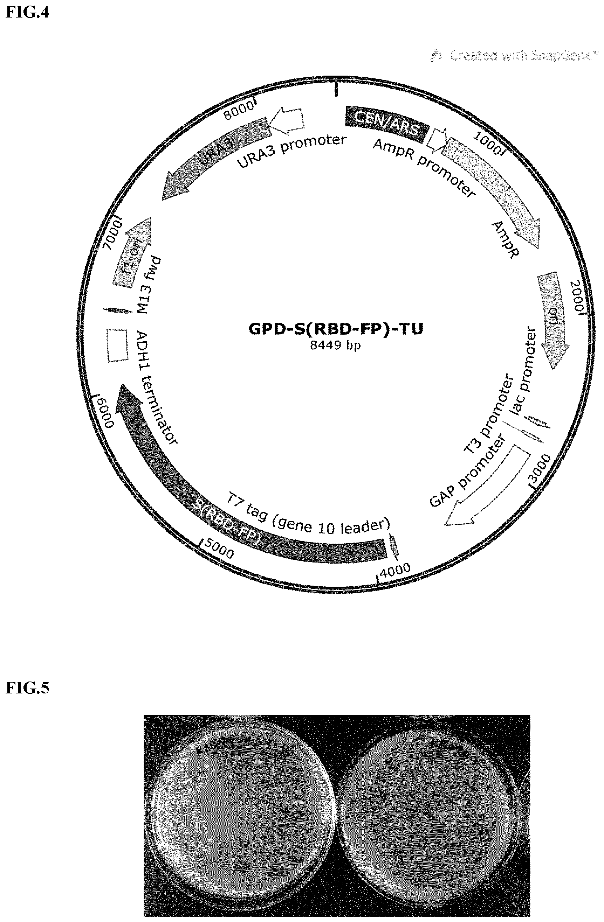

A vector *GPD-S(RBD-FP)-TU* was subjected to enzyme digestion with *Bsa*I*,* and meanwhile homologous arm plasmids (URR1 and URR2) and a selective tag plasmid (LEU) were subjected to enzyme digestion with *Bsm*B I. Homologous arms URRs, the selective tag LEU and a transcriptional unit *GPD-S(RBD-FP)-TU* were spliced according to a specific prefix and suffix sequence by referring to experimental steps of a Dai research group^{[6]}, and were linked overnight with T4 ligase at 16□, and a spliced product (shown in FIG. 4) was used for yeast transformation.

### (2) Construction of recombinant saccharomyces cerevisiae strain ST1814G-S(RBD-FP)

① Strain activation: a single colony ST1814G was picked and inoculated into 3 mL of a YPD liquid medium to be cultured overnight at 30°C and 220 rpm. A bacteria solution cultured overnight was transferred into 5 mL of a fresh YPD medium at a ratio of 1: 50, so that an initial OD is 0.1-0.2, and the medium was cultured at 30°C and 220 rpm till OD600 was 0.5-0.8. The medium was centrifuged at 2500 rpm for 5 minutes, bacteria of 5 mL of suspension were collected, cells were washed with 1 mL of sterile water, and a supernatant was removed.
② Yeast transformation: 100 µL of 0.1 M lithium acetate was added into the centrifuged bacterium precipitates, the cells were resuspended, and were centrifuged at 12000 rpm for 20 seconds, and a supernatant was removed. 50 µL of 0.1 M lithium acetate was added thereto, cells were resuspended, and were centrifuged at 12000 rpm for 20 seconds, and the bacteria were collected. Then 240 µL of 50% PEG4000, 36 µL of 1 M lithium acetate, 100 µg of salmon sperm DNA and 2 µg of fragment DNA were sequentially added into a centrifugal tube and shaken vigorously till they were completely mixed well. The mixture was centrifuged at 30 □ and 200 rpm for 30 minutes, at 42□ for 25 minutes and at 6000 rpm for 15 seconds, bacteria were collected, a transformation solution was removed, 1 mL of the YPD liquid medium was added for incubation at 30□ for 2 hours, and was centrifuged at 2500 rpm for 5 minutes, bacteria were collected, cells were resuspended with 50 µL of deionized water, the cells were blown and pipetted and mixed well as moderate as possible, and the bacteria were coated on a surface of an SD-leu solid medium and grew in an incubator at 30□ for 2-3 days till typical colonies were formed. A single colony was picked, streaked and purified on an SD-leu plate and synchronously inoculated into 3 mL of the YPD liquid medium to be cultured overnight at 30 □ and 220 rpm for genome validation.
(3) Genotype validation of strain ST1814G-S(RBD-FP)

Yeast growing in the SD-leu medium was selected, and a genome thereof was extracted to validate whether the gene *S(RBD-FP)* was successfully integrated into the genome or not through PCR. An extraction method of the yeast genome was performed by referring to documents ^{[10]}. 100 µL of a cultured bacteria solution was taken and centrifuged at 6000 rpm for 1 minute, a supernatant was removed, cells were resuspended with 100 µL of lysate (200 mM LiOAc, 1% SDS) and incubated at 70°C for 5 minutes, 300 µL of absolute ethanol was added for mixing them well, the mixture was centrifuged at 15000 g for 3 minutes, and cell debris was collected. After washing with 70% ethanol, 50-100 µL of ddH₂O was added for resuspending precipitates. After centrifuging at 15000 g for 15 seconds, a supernatant was pipetted as a genome template for PCR amplification. A PCR amplification system was as follows:

| | |
|---|---|
| Genome DNA | 1 µL |
| S-F 331 (10 µM) | 1 µL |
| S-R 524 (10 µM) | 1 µL |
| 2*Mix Buffer (P111-01, Vazyme) | 10 µL |
| ddH₂O | To 20 µL |

Amplification was performed by using the following PCR procedures:

| | | | |
|---|---|---|---|
| 95°C | 1 min | 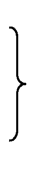 | 30 cycles |
| 95°C | 15 s | | |
| 56°C | 15 s | | |
| 72°C | 30 s | | |
| 72°C | 5 mins | | |

PCR amplification was performed with genome DNA as a template, and meanwhile the plasmid GPD*-S(RBD-FP)*-TU was taken as a positive control and ddH₂O was taken as a negative control.

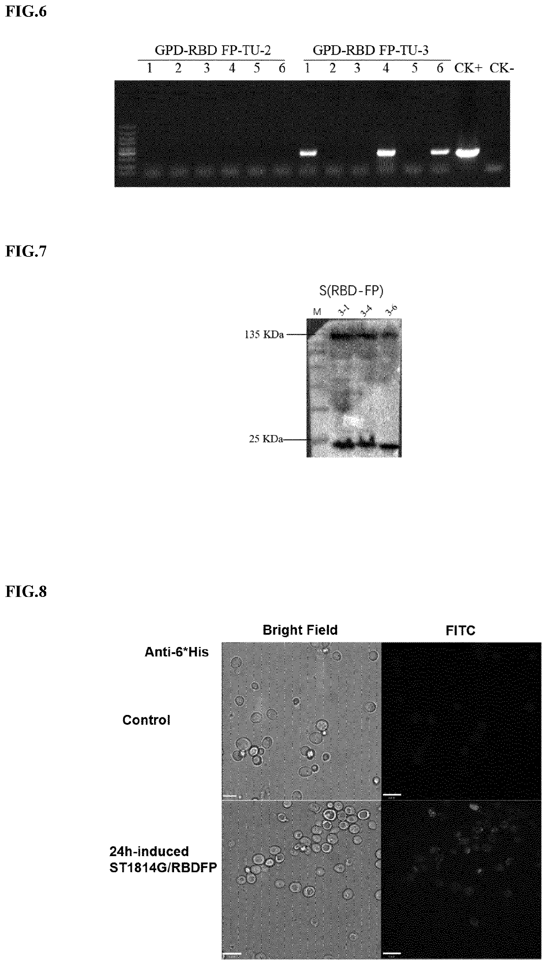

Experimental results were shown in FIG. 5, *Escherichia coli* transformants No. 2 and No. 3 of the plasmid GPD*-S(RBD-FP)*-TU transformed yeast cells after enzyme digestion and linkage, and although both of them could grow on the Sd-leu selective medium, only the No. 3 plasmid could obtain the recombinant yeast strain ST1814G-S(RBD-FP) with a correct genome. The correct recombined yeast was numbered with No. 1, No. 4 and No. 6 (shown in FIG. 6).
④ Phenotype validation of strain ST1814G-S(RBD-FP)

As a C-terminal, inserted into the fragment S(RBD-FP), of the vector GPD*-S(RBD-FP*)-TU had a His tag, an expression of a target gene *S(RBD-FP)* was tested by an anti-His antibody for Western blot, and display of the protein was observed by using a laser confocal microscope.

### Western Blot:

2 mL of a YPD culture solution for 48 hours was collected and centrifuged at 6000 rpm for 1 minute, bacteria were collected, acid-pickling glass beads were added thereto, the bacteria were resuspended with 60 µL of PEB buffer, wall breaking was performed five times by a glass bead method, 15 µL of 5×SDS loading buffer was added, the bacteria were left to stand in a boiling water bath for 5 minutes, and were centrifuged at 4°C and 12000 rpm for 10 minutes, a supernatant was carefully pipetted, and 8 µL of the supernatant was used for 12% SDS-PAGE electrophoresis.

Protein separation gel was transferred to a PVDF membrane with a size the same as that of the gel at 300 mA for 100 minutes by a wet transfer method after SDS-PAGE electrophoresis was completed; the PVDF membrane was blocked with 5% skim milk for 1 hour at room temperature after membrane transfer; the membrane was completely immersed in a mouse anti-His monoclonal antibody (HT501, Transgene) diluted with 5% BSA at a ratio of 1: 2000 and incubated at 4°C overnight; a primary antibody was recovered, and the PVDF membrane was rinsed three times with TBST buffer, each time for 10 minutes; a goat anti-mouse HRP-labeled secondary antibody diluted with 5% of BSA at a ratio of 1: 5000 (LK2003, Sungene Biotech) was added, and incubation was performed at room temperature for 1 hour; the PVDF membrane was rinsed three times with the TBST buffer; and a chemiluminescence chromogenic substrate (34075, ThermoFisher) was dropwise added to a front surface of the PVDF membrane in the dark, and exposure was performed by a Bio-rad chemiluminescence imager to observe a protein expression.

### Immunofluorescence:

500 µL of ST1814G-S(RBD-FP) bacteria solution induced for 48 hours was collected and centrifuged at 4000 rpm for 5 minutes, and bacteria were collected; ST1814G induced for 48 hours was taken as a negative control; the bacteria were washed three times with 500 µL of PBST and centrifuged at 4000 rpm for 5 minutes, and a supernatant was removed; the bacteria were resuspended with an 6*His-Tag antibody diluted with 200 µL of PBST at a ratio of 1: 2000 respectively, and spin binding was performed at 4°C for 1 hour; a primary antibody was recovered; the bacteria were washed three times with 500 µL of PBST and centrifuged in the similar way, and a supernatant was removed; an FITC-labeled goat anti-mouse secondary antibody diluted with 200 µL of PBST at a ratio of 1: 5000 was added, and incubation was performed at 37°C for 30 minutes in the dark and then, the secondary antibody was recovered; the bacteria were washed three times with 500 µL of PBST and centrifuged in the similar way, and a supernatant was removed; and the bacteria were resuspended with 50 µL of PBS, 10 µL of bacteria solution was taken and dropwise added to a clean glass slide and covered with a cover slip, the cover slip was fixed by coating nail polish around the cover slip, and the bacteria solution was observed under a laser confocal microscope.

Results were shown in FIG. 7, and recombinant yeast strains No. 1, No. 4 and No. 6 all could express the S protein. A theoretical value of a molecular weight of the S protein was 78 KDa, however, an actual observation result showed two bands of 135 KDa and 23 KDa. The S protein has a plurality of glycosylation sites, so that the increase of the molecular weight may be caused by glycosylation. The decrease of the molecular weight may be caused by digestion with protease. Immunofluorescence results were shown in FIG. 8, and no fluorescence was shown in the control group ST1814G (above FIG. 8), whereas obvious green fluorescence was shown in the experimental group ST1814G-S(RBD-FP) (below FIG. 8), indicating that the S protein was successfully expressed and positioned on surfaces of the recombinant saccharomyces cerevisiae cells.
(5) Growth and protein expression rule of strain ST1814G-S(RBD-FP)

A single colony of a transformant No. 4 of the strain ST1814G-S(RBD-FP) was taken and inoculated to 20 mL of a YPD liquid medium, 2 mL of the medium was sampled at an interval of 24 hours for OD₆₀₀ determination, the volume of bacteria was adjusted to be consistent based on the minimum value of the bacterial volume at an initial time point, and protein samples at different time points were prepared for Western blot test.

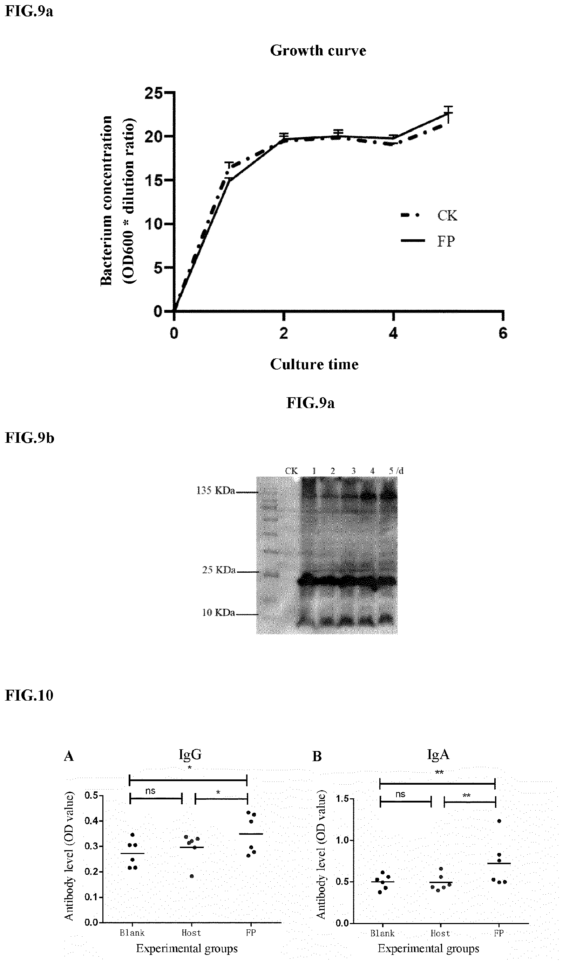

Results were shown in FIG. 9, and it was analyzed from a growth curve of FIG. 9-a that the expression of the S(RBD-FP) truncated protein had no influence on growth of the bacteria, the growth trend of the bacteria was consistent with that of the control group, and the volume of the bacteria was gradually increased over the extension of the culture time. Meanwhile, the expression level of the protein was also increased over the extension of the culture time, in which the expression level was the highest on Day 5 of culture (FIG. 9-b).

### Embodiment 3. Preparation of recombinant yeast for S protein of coronavirus

A No. 4 strain ST1814G-S(RBD-FP) was taken as a seed, a single colony was taken and inoculated into 20 mL of a YPD liquid medium to be cultured overnight at 30°C, a seed solution cultured overnight was taken to be diluted at a ratio of 1: 100, inoculated into 1 L of fresh YPD liquid medium, after cultured for 3 to 4 days, the medium was centrifuged, bacteria were collected and washed once with PBS, and recombinant strain dry powder was prepared after the bacteria were subjected to vacuum freeze-drying.

### Embodiment 4. Application of recombinant yeast strain for S protein of coronavirus

SPF-level BALB/c mice were divided into three groups with six mice in each group, each was fed with 10⁷cfu of a No. 4 yeast strain ST1814G-S(RBD-FP) on Day 1, Day 6, Day 11 and Day 23 respectively, and a blank yeast strain ST1814G was taken as a control. Blood was sampled from eyeballs after the fourth immunization for test of levels of IgG and IgA antibodies.

An RBD domain for prokaryotically expressed S protein of coronavirus was designed in a test, the purified protein was obtained, the antibody was prepared, and the purified RBD protein, goat anti-mouse IgG and IgA-HRP enzyme conjugates and indirect ELISA were used for testing the levels of IgG and IgA.

Results were shown in FIG. 10, the levels of S protein-specific IgG (FIG. 10A) and IgA (FIG. 10B) in serum of the mice in the group fed with the No. 4 recombinant yeast ST1814G were significantly increased, indicating that the recombinant saccharomyces cerevisiae strain could well stimulate organism's body fluid immunization and mucosal immunization, and could serve as a novel immune protection preparation, and provide options for prevention and control of the coronavirus.

Although the content of the present invention is described with reference to the above embodiments, implementations of the present invention are not limited by the above embodiment. The scope of the present invention is limited by the appended claims, and any other changes or modifications made within the limited scope all should be equivalent substitutes that are included in the protection scope of the present invention.

### References:

[1]. Meyerholz, D.K., A.M. Lambertz and P.B. McCray, Dipeptidyl Peptidase 4 Distribution in the Human Respiratory Tract. The American Journal of Pathology [J]. 2016. 186(1): p. 78-86.
[2]. Zhou, P., et al., A pneumonia outbreak associated with a new coronavirus of probable bat origin [J]. Nature, 2020.
[3]. Ge, X., et al., Isolation and characterization of a bat SARS-like coronavirus that uses the ACE2 receptor [J]. Nature, 2013. 503(7477): p. 535-538.
[4]. Tian, X., et al., Potent binding of 2019 novel coronavirus spike protein by a SARS coronavirus-specific human monoclonal antibody [J]. Emerging Microbes & Infections, 2020. 9(1): p. 382-385.
[5]. Chen, W. and G. Georgiou, Cell□ Surface display of heterologous proteins: From high□throughput screening to environmental applications [J]. Biotechnology and Bioengineering, 2002. 79(5): p. 496-503.
[6]. Guo, Y., et al., YeastFab: the design and construction of standard biological parts for metabolic engineering in Saccharomyces cerevisiae [J]. Nucleic Acids Research, 2015. 43(13): p. e88-e88
[7]. Huang Jinhai, Method for preparing P30 and P54 yeast vaccines for African swine fever virus [P]. Chinese patent No. 201911286530.6, 2019. 12. 13
[8]. Yan, R., et al., Structural basis for the recognition of the SARS-CoV-2 by full-length human ACE2. Science (New York, N.Y.), 2020: p. eabb2762.
[9]. Song, W., et al., Cryo-EM structure of the SARS coronavirus spike glycoprotein in complex with its host cell receptor ACE2. PLOS Pathogens, 2018. 14(8): p. e1007236.
[10]. Lõoke, M., et al., Extraction of genomic DNA from yeasts for PCR-based applications. Biotechniques, 2011. 50(5): p. 325-328.

## Claims

1. A recombinant yeast for expressing an S protein of coronavirus, ST1814G-S (RBD-FP), containing amino acids of the S protein at positions 16 to 1035.

2. A truncated S protein of coronavirus, **characterized in that** the truncated S protein is preferably amino acids at positions 300 to 1000, with a sequence characteristic of SEQ ID No. 1, containing an RBD domain at positions 330 to 521 and an FP fusion peptide at position 816 to 833.

3. A gene for expressing the truncated protein according to claim 2, **characterized in that** the gene contains an RBD domain of a spike protein S, namely basic groups at positions 991 to 1563 and an FP domain, namely basic groups at positions 2449 to 2499, with a nucleotide sequence of SEQ ID No. 2.

4. Construction of a plasmid *GPD-S(RBD-FP)-TU* of the recombinant yeast according to claim 1, **characterized in that** the plasmid has a sequence characteristic of SEQ ID No. 3 and consists of the gene fragments according to claim 3 and a POT-GPD-TU vector.

5. A method for preparing a recombinant yeast for an S protein of coronavirus, **characterized by** comprising the following steps: integrating a complete transcriptional unit *GPD-S(RBD-FP)-TU* of a truncated S protein body constructed in vitro into a yeast genome through homologous recombination, displaying the S protein on a surface of a yeast cell by an Aga1-Aga2 surface display system to obtain an S protein surface display type recombinant yeast strain ST1814G-S(RBD-FP), and preparing the oral recombinant yeast by using the obtained strain.

6. The method for preparing the recombinant yeast for the S protein of the coronavirus according to claim 5, **characterized by** specifically comprising the following steps:
(1) PCR amplification of an encoding gene of the spike protein S of the SARS-CoV-2: synthesizing an S gene by referring to a virogene sequence NC_045512.2 of the SARS-CoV-2, with a sequence characteristic of SEQ No. 2; and designing primers to amplify the encoding gene *S(RBD-FP)* of the S protein for yeast vector linkage with a plasmid pcDNA3.1-CoV-S as a template;
(2) tandem between an Aga2 gene and an encoding sequence *S(RBD-FP)* of the spike protein S of the SARS-CoV-2: linearizing the POT-GPD-TU vector by single enzyme digestion with *Bam*HI*,* seamlessly cloning and linking S gene fragments to a surface display expression vector GPD-POT-TU to obtain a recombinant plasmid GPD-*S(RBD-FP)*-TU with a sequence characteristic of SEQ No. 3, transforming the recombinant plasmid into *E.coli* DH5a, and performing PCR and sequencing validation by using S gene test primers to obtain a positive clone; and
(3) construction of an S protein recombinant yeast strain: performing enzyme digestion and splicing on the recombinant plasmid GPD-*S(RBD-FP)*-TU, homologous arms URRs and an encoding sequence of a screening tag Leu to obtain a complete recombinant gene containing an S gene sequence, transforming the recombinant gene into a saccharomyces cerevisiae genome, obtaining a recombinant strain after screening with an auxotrophy type plate, testing a gene level by using the test primers, and validating a protein expression level by Western blot and immunofluorescence.

7. Application of a recombinant yeast for an S protein of coronavirus in preparation of a drug against the Coronavirus.
